# EUROPEAN PATENT APPLICATION

(11) **EP 1 260 518 A1**
(43) Date of publication of application: **27.11.2002**
(21) Application number: 01908239.5
(22) Date of filing: 02.03.2001
(51) Int. Cl.: C07K 1/20, C07K 1/18

(54) **METHOD OF SEPARATING AND PURIFYING PROTEIN**

(30) Priority: 02.03.2000 JP 2000057672
(71) Applicant: KYOWA HAKKO KOGYO CO., LTD., Chiyoda-ku, Tokyo 100-8185 (JP)
(72) Inventor: UCHIDA, Kazuhisa, Tokyo Research Laboratories, Machida-shi, Tokyo 194-8533 (JP); YAMASAKI, Motoo, Tokyo Research Laboratories, Machida-shi, Tokyo 194-8533 (JP)
(74) Representative: VOSSIUS & PARTNER
(86) International application number: JP0101610
(87) International publication number: WO01064711

(57) **Abstract**

A method for the production of a highly purified protein at a moderate price which can be provided as a medicament is provided, by constructing a system for purifying a protein having its desired property efficiently and in a high yield independent of physicochemical properties inherent in the protein having a desired property such as isoelectric point and hydrophobicity strength.

## Description

### TECHNICAL FIELD

The present invention relates to a method for separating impurities and a protein having a desired property coexisting in a solution, and purifying the protein having a desired property.

### BACKGROUND OF THE INVENTION

In the production of protein medicaments, the protein is generally purified by a process in which plural chromatography are combined as shown in, for example, Japanese Published Examined Patent Application No. 288897/90 and the like. The object for carrying out the above purification includes improvement of the purity of a desired protein; elimination of impurities such as endotoxin which is a pyrogen containing an acidic substance lipopolysaccharide as the main component, proteins, nucleic acid and the like derived from host animal cells, *Escherichia coli* and the like, fetal bovine serum-derived proteins, serum albumin, transferrin and insulin which are added at culturing and degradation products, analogues and the like of the desired protein thereof; and the like.

Kinds of generally used chromatography for adsorbing a protein and problems are described as follows.

As a method using specific binding of a protein, affinity chromatography [*Affinity Chromatography* (Pharmacia, 1998)] is known. As the characteristic of affinity chromatography, a desired protein can be selectively and efficiently purified from a complex mixed solution of proteins such as a culture medium, a tissue extract or the like.

However, since it is necessary that a substance which specifically binds to the desired protein is present, the affinity chromatography cannot be used for the purification of all proteins. Also, since binding of the desired protein to the resin for affinity chromatography is too strong, the recovery rather becomes low in some cases. Also, since the elution conditions are severe in many cases, the desired protein may be denatured and the activity of the protein may disappear. In addition, there are many problems such as washing away of a ligand, adsorption of non-specific substances other than the desired protein to a ligand or a ligand-linked carrier and the like during the purification process. Also, resins for affinity chromatography are more expensive than other chromatography carriers.

Protein A, protein G or the like is used as a ligand for purifying antibodies such as immunoglobulin (IgG). However, for example, since protein A has a property to link to the Fc region of IgG of various animals, a fetal bovine serum-derived IgG other than the desired IgG and the like added to a medium also links to protein A. Also, since a culture medium is contaminated with heavy chains of a monomer having an incomplete Fc region, IgG fragments digested with a protease and the like, and serum proteins such as albumin which is nonspecific adsorption substance and the like, the purification degree becomes low so that the purity of a solution containing the desired protein is reduced. Also, in the purification step, the antibody adsorbed onto a protein A column is generally eluted with an acidic eluate of pH 4 or less. When the pH of the eluate is 4 or less, there is a danger in that the antibody is denatured or inactivated and thereby generates antigenicity when administered into the living body.

When there is no specific ligand for the desired protein, the desired protein is expressed as a fusion protein fused with other protein and the fusion protein is subjected to chromatography for using a carrier having a ligand which binds to the other protein. For example, the desired protein can be obtained by fusing with a specific protein such as glutathione S synthetase (GST) and expressing the fused protein, followed by subjecting to chromatography using the GST-fused protein as the ligand and then cutting out the GST. However, since the safety of GST protein has not been established, the method is inappropriate in view of safety and the like because it may be contaminated during the purification process of protein medicaments.

As is described, e.g., in *Fundamental Experiments of Proteins and Enzymes* (Nanko-do, 1981), *Protein Purification Methods* (IRL PRESS, 1989) or *Perfusion Chromatography* (Perceptive Biosystems Inc., 1997), ion exchange chromatography is carried out under mild protein adsorption reaction conditions so that it is an effective method for purifying proteins in view of obtaining proteins with a high recovery ratio without losing physiological activities.

Since the ion exchange chromatography is a separation method based on the difference in electric charges of respective components, the pH of the solution has a close relationship to the isoelectric point of the protein having a desired property. The ion exchanger includes a cation exchanger and an anion exchanger. When the solution pH is lower than the isoelectric point of the protein having a desired property, the total charge of the protein becomes positive so that the protein is adsorbed onto a cationic exchanger, whereas, when the solution pH is higher than the isoelectric point of the protein having a desired property, the total charge of the protein becomes negative so that the protein is adsorbed onto an anionic exchanger.

Also, in order to adsorb a desired protein onto a resin, it is recommended to keep the pH of the initiation buffer apart from the isoelectric point of the protein by a pH of at least 1 (*Ion Exchange Chromatography Principles and Methods,* Pharmacia). Thus, the primary object of the ion exchange chromatography is to adsorb a desired protein onto a resin and recover it with a high recovery yield, by taking the isoelectric point of the desired protein, the pH of the solution, characteristics of the carrier and the like into consideration. Accordingly, it is necessary to carry out separation of impurities and the desired protein within the pH range, so that quality of the separation must be a secondary object.

For example, a case in which the desired protein is an antibody is described.

Although isoelectric point of antibodies varies depending on the post-translational modification and the like, the pI value is 5 to 8. For example, isoelectric points of mouse monoclonal antibodies described on page 205 of *COMMERCIAL PRODUCTION OF MONOCLONAL ANTIBODIES* (MARCEL DEKKER INC., 1987) are pI 7.5, 7.3, 7.1, 6.9, 6.8, 6.7 and 6.6 due to post-translational modification, and the isoelectric point of rabbit antiserum described in *Immune Experiments* (Nishimura Shoten, 1985) is broadly distributed between pI 5 and 8.

However, since isoelectric points of proteins derived from antibody-producing host cells or sera, serum-originated proteins or substitutes for the serum-originated proteins added to the medium are also within the pI range of 4 to 7 (*GEL ELECTROPHORESIS: PROTEINS BIOS SCIENTIFIC PUBLISHERS* (1993), *Fundamental Experiments of Proteins and Enzymes* (Nanko-do, 1981), they are overlapped with the isoelectric points of antibodies. As a result, their elution pattern in the purification becomes the same and the separation becomes difficult. Particularly, since isoelectric points of albumin and transferrin to be added in large amounts to a serum-free medium are around pI 4.5 to 6 and pI 5 to 6, respectively, depending on the kind of desired antibody, its elution pattern becomes the same as these impurity proteins in the ion exchange chromatography, so that the ion exchange chromatography is not desirable from the viewpoint of purification efficiency and purity improvement. Such phenomena are general in the purification of not only antibodies but also proteins using the ion exchange chromatography.

As is described in, e.g., *Fundamental Experiments of Proteins and Enzymes* (Nanko-do, 1981) or *Perfusion Chromatography* (Perceptive Biosystems Inc., 1997), hydrophobic chromatography is used in the purification of proteins because it is carried out under mild binding reaction conditions and its recovery yield and adsorption strength are generally excellent. The hydrophobic chromatography is a method for the separation of proteins using a property in which interaction between hydrophobic groups of a resin and hydrophobic moieties of a protein is different in each protein. Also in the hydrophobic chromatography, as described in, e.g., *Hydrophobic Interaction Chromatography* (Pharmacia Biotech 1993), it is recommended to carry out determination of elution conditions, selection of resins and the like of the chromatography by taking elution position and recovery yield into consideration based on the hydrophobicity inherent in the desired protein, so that this is not necessarily a method which can separate the desired protein from impurities.

### DISCLOSURE OF THE INVENTION

When a protein is purified using a chromatography, the purification method is constructed based on the physicochemical property of the protein such as its isoelectric point or hydrophobicity. In this case, when the coexisting impurities have similar physicochemical properties to those of the desired protein, it causes problems such as inhibition of purity improvement of the desired protein, reduction of its yield, reduction of its quality, increase in its purification cost and the like.

An object of the present invention is to provide a method for producing a highly purified protein at a moderate price which can be provided as a medicament, by constructing a method for purifying the desired protein efficiently and with a high yield while keeping its desired property.

The present invention relates to the following (1) to (10):
(1) A method for separating and purifying a protein, which comprises:
   changing a physicochemical property of a protein while having a desired property of the protein, and then
   separating and purifying the protein.
(2) The method according to (1), wherein the physicochemical property is at least one selected from an isoelectric point and hydrophobicity of the protein.
(3) The method according to (2), wherein the isoelectric point is pI 7 or more.
(4) The method according to (2), wherein the isoelectric point is pI 4.5 or less.
(5) The method according to (2), wherein the hydrophobicity is similar to or higher than that of immunoglobulin G.
(6) The method according to (2), wherein the hydrophobicity is such a degree that the protein can be adsorbed onto a phenyl sepharose resin in a buffer comprising 1 mol/l ammonium sulfate.
(7) The method according to (2), wherein the hydrophobicity is similar to or lower than that of serum albumin.
(8) The method according to (1), wherein the method of changing a physicochemical property of the protein is deletion, substitution or addition of an amino acid constituting the protein, or preparation of a fusion protein with other protein.
(9) The method according to any one of (1) to (8), wherein the method of separating and purifying the protein which has a desired property comprises a step of using at least one chromatography of hydrophobic chromatography and ion exchange chromatography.
(10) The method according to any one of (1) to (9), wherein the protein is an antibody.

The present invention relates to a method for purifying a protein while having a desired property, by changing a physicochemical property of a desired protein while having a desired property of the protein and then separating the protein having the desired property from impurities coexisting in the solution. The protein having the desired property may be any protein which satisfies the object of those who use the protein. That is, it is enough so long as a property desired by the users is maintained among the original properties of the protein.

Examples of the desired property include a three-dimensional structure of the protein, a physiological activity of the protein, affinity for a specified substance, stability in blood and the like.

As the physicochemical properties of the protein, isoelectric point, hydrophobicity and the like are included. That is, to change a physicochemical property is to adjust the isoelectric point to pI 7 or more or pI 4.5 or less, or to adjust the hydrophobicity to similar to or higher than the hydrophobicity of immunoglobulin G or to similar to or lower than the hydrophobicity of serum albumin.

Examples of the impurities include endotoxin which is a pyrogen containing an acidic substance lipopolysaccharide as the main component, proteins, nucleic acid, lipids and saccharides derived from host cells such as animal cells, *Escherichia coli* and the like, fetal sera of mammals, proteins of the sera, plant proteins, serum albumin, transferrin or insulin to be added to a culture medium, degradation products and analogues of the desired protein, and the like.

Accordingly, in order to purify a protein having a desired property efficiently with high purity from a solution containing the desired protein such as a cell culture medium, tissue extract, ascitic fluid or the like, the protein having a desired property can be separated from impurities by changing physicochemical property of the protein such as isoelectric point, hydrophobicity or the like from that of the impurities existing in the solution without changing desired property of the protein.

The desired protein may be any protein which is present in a disrupted solution of tissues or cells in the living body, serum, pleural effusion, ascitic fluid, ophthalmic fluid, urine or a cell culture medium. Examples include various cytokines such as interleukin, interferon, *etc.*; serum proteins such as antibodies, *etc.;* various enzymes such as protease, proteins which inhibit the enzymes, *etc.*; and the like. Also, the proteins may be expressed by genetic engineering techniques.

An example of the method for expressing the proteins by genetic engineering techniques is a method in which a cDNA encoding a protein having a desired property is inserted into downstream of the promoter of an appropriate vector and a protein-expressing cell obtained by introducing the thus constructed recombinant vector into a host cell is cultured in an appropriate medium to thereby produce the protein having a desired property intracellularly or in the culture supernatant [*Molecular Cloning,* 2nd edition, Cold Spring Harbor Lab. Press, New York (1989); hereinafter referred to as "*Molecular Cloning,* 2nd edition"].

Accordingly, methods for changing physicochemical properties of a desired protein are specifically described as follows.

Examples of the methods for changing physicochemical properties of a protein such as isoelectric point, strength of hydrophobicity and the like include a method in which the protein is modified by deleting, substituting or adding an amino acid(s) as the primary structure of the protein, a method in which it is expressed as a fusion protein with other protein, and the like.

The method for deleting, substituting or adding an amino acid(s) as the primary structure of a protein having a desired property can be carried out by introducing site-specific mutation into a DNA encoding the protein using the site-directed mutagenesis method described in *Nucleic Acids Research,* 10, 6487 (1982); *Proc. Natl. Acad. Sci.*, *USA,* 79, 6409 (1982); *Gene*, 34, 315 (1985); *Nucleic Acids Research,* 13, 4431 (1985); *Proc. Natl. Acad. Sci., USA,* 82, 488 (1985); and the like.

As the method for expressing a fusion protein with other protein, the full length of a protein having a desired property or a partial fragment thereof can be produced as such or as a fusion protein intracellularly or in a culture supernatant by inserting a DNA encoding a fusion protein into downstream of the promoter of an appropriate vector and culturing a protein-expressing cell obtained by introducing the thus constructed recombinant vector into a host cell in an appropriate medium (*Molecular Cloning,* 2nd edition).

The method for changing the hydrophobicity includes a method for producing a mutated protein having high hydrophobicity or a method producing a mutated protein having low hydrophobicity.

The method for producing a mutated protein having high hydrophobicity is a method in which an amino acid residue(s) in the primary structure of the desired protein are substituted with other amino acid(s) having higher hydrophobicity than that of the original residue(s). So long as the protein has the desired property, it is possible to produce a mutated protein having high hydrophobicity by adding an amino acid(s) having high hydrophobicity or deleting an amino acid(s) having low hydrophobicity.

The method for producing a mutated protein having low hydrophobicity is a method in which an amino acid residue(s) in the primary structure of the desired protein are substituted by other amino acid(s) having lower hydrophobicity than that of the original residues. So long as the protein has the desired property, it is possible to produce a mutated protein having low hydrophobicity by adding an amino acid(s) having low hydrophobicity or deleting an amino acid(s) having high hydrophobicity.

The method for changing the isoelectric point includes a method for the preparation of a basic mutated protein or an acidic mutated protein, and the like.

A basic mutated protein can be produced by substituting a non-basic amino acid(s), among the original amino acid residues in the primary structure of the desired protein, with a basic amino acid(s), or when the desired property should be maintained, by adding a basic amino acid(s) or deleting an acidic amino acid(s).

When an acidic mutated protein is produced, the acidic mutated protein can be prepared by substituting a non-acidic amino acid(s), among the original amino acid residues in the primary structure of the desired protein, with an acidic amino acid(s), or when the desired property should be maintained, by adding an acidic amino acid(s) or deleting a basic amino acid(s).

In antibodies among proteins, the heavy chain (hereinafter referred to as "H chain") Fc region of a human antibody has high basic property. Accordingly, a basic fusion protein can be obtained by producing a fusion protein of a desired protein with the Fc region by genetic engineering techniques.

A basic antibody can be obtained by substituting H chain Fc region or light chain constant region (hereinafter referred to as "L chain C region") of a monoclonal antibody produced by a hybridoma by human H chain Fc region or human L chain C region.

The physicochemical property is changed by the above methods and a protein having a desired property can be separated and purified efficiently using general protein separation purification methods.

For example, a protein having a desired property and impurities can be separated using the following hydrophobic chromatography, ion exchange chromatography and gel filtration chromatography alone or in combination.

When the hydrophobicity of a protein having a desired property is higher than that of contaminated proteins, a solution containing the protein having a desired property is mixed with a buffer containing ammonium sulfate and then applied to a hydrophobic chromatography column, and the contaminated proteins are passed through and the protein having a desired property is adsorbed onto the column. The protein having a desired property and contaminated proteins can be separated by eluting the protein having a desired property while decreasing ammonium sulfate concentration in the eluate.

When the hydrophobicity of a protein having a desired property is lower than that of contaminated proteins, the protein having a desired property and contaminated proteins can be separated by mixing a solution comprising the protein having a desired property with a buffer comprising ammonium sulfate, applying the mixture to a hydrophobic chromatography column, adsorbing the contaminated proteins onto the hydrophobic chromatography column and then recovering the protein having a desired property passed through the column.

The resin used in the hydrophobic chromatography may be any resin for hydrophobic chromatography which is commercially available. Examples include Phenyl Sepharose 6FF, Phenyl Sepharose HP, Butyl Sepharose 4F, SOURCE 15 PHE, STREAMLINE Phenyl (manufactured by Pharmacia), Ether-TOYOPEARL 650, Butyl- TOYOPEARL 650 (manufactured by TOSOH) and the like.

An example of the method using it is shown below.

First, a resin is packed in a column, and then components, such as endotoxin which is a pyrogen, strongly binding to the resin are washed with 0.1 to 1N, preferably 0.5 N of sodium hydroxide. Sterilization is carried out against microorganisms and the like which are generated in the column. To a protein solution containing a protein having a desired property, 0 to 1.5 mol/l solid or liquid ammonium sulfate is added.

When the protein having a desired property is adsorbed onto the column, for example, Phenyl-Sepharose FF resin is used and the ammonium sulfate concentration is adjusted to 1 mol/l, preferably 0.8 mol/l. Also, the eluate used in the chromatography includes a buffer such as 10 to 100 mmol/l citrate-glycine buffer, 10 to 100 mmol/l sodium phosphate buffer and the like. The pH is adjusted to a value of approximately from 5 to 8, preferably around pH 7. After thorough equilibration of the column with an initial stage eluate such as 10 to 100 mmol/l citrate-glycine buffer, 10 to 100 mmol/l sodium phosphate buffer or the like containing ammonium sulfate, a sample such as an ammonium sulfate-treated culture medium or the like is applied to the column. When the protein having a desired property passes through the column, the passed fraction is optionally subjected to treatments such as concentration, desalting and the like and then subjected to the next step. When the protein having a desired property adsorbs onto the column, the initial stage eluate or an initial stage eluate by which the protein having a desired property is not eluted is mixed with a final eluate and applied to the column, and components unadsorbed onto the column are washed. Ultraviolet ray absorption of the eluted fractions is measured, and when the ultraviolet ray absorption becomes constant, the elution is carried out by a stepwise method or a gradient method using the final eluate as eluate. A fraction containing the protein having a desired property can be detected by a usual protein detection method such as ultraviolet ray absorption, electrophoresis or activity measurement of the protein.

When the isoelectric point of the protein having a desired property is adjusted to a level higher than that of contaminated proteins, a solution containing the protein having a desired property is diluted, or the salt concentration of elution fractions is reduced to an appropriate concentration by dialysis, and the sample is applied to a column packed with a cation exchange resin and the protein having a desired property is adsorbed onto the column while allowing the contaminated proteins to pass through the column. The salt concentration in the eluate is increased and applied to the column onto which the protein having a desired property is adsorbed, and then the protein having a desired property is eluted.

When the isoelectric point of the protein having a desired property is adjusted to a level lower than that of contaminated proteins, a solution containing the protein having a desired property is diluted, or the salt concentration of elution fractions is reduced to an appropriate concentration by dialysis, and the sample is applied to a column packed with an anion exchange resin, the contaminated proteins are adsorbed onto the column and the protein having a desired property is passed through the column and recovered. In this case, cation exchange chromatography may be used in combination.

The resin used in the cation exchange chromatography may be any resin for cation exchange chromatography which is commercially available. Examples include SP Sepharose FF, CM Sepharose FF, SP Sepharose HP, SOURCE 305, STREAMLINE SP (manufactured by Pharmacia), CM-TOYOPEARL 650, SP- TOYOPEARL 650 (manufactured by TOSOH) and the like.

The resin used in the anion exchange chromatography may be any resin for anion exchange chromatography which is commercially available. Examples include Q Sepharose FF, DEAE Sepharose FF, Q Sepharose HP, SOURCE 30Q, STREAMLINE DEAE (manufactured by Pharmacia), DEAE- TOYOPEARL 650, QAE-TOYOPEARL 550 (manufactured by TOSOH) and the like.

An example of the method using it is shown below.

First, a resin is packed in a column, and then components, such as endotoxin which is a pyrogen, strongly binding to the resin are washed with 0.1 to 1 N, preferably 0.5 N of sodium hydroxide. Sterilization is carried out against microorganisms and the like which are generated in the column. By controlling the pH, salt concentration or kind of the salt and the like in the fractions containing a protein having a desired property, conditions under which the protein having a desired property passes through the column or adsorbs onto the column are selected.

The eluate includes a buffer such as 10 to 100 mmol/l citrate-glycine buffer, 10 to 100 mmol/l sodium phosphate buffer and the like, and the pH of the eluate is adjusted to approximately 5 to 8, preferably around 7. After thorough equilibration of the column with an initial stage eluate, a sample such as a culture medium or the like treated under the conditions determined in the above is applied to the column.

When the protein having a desired property passes through the column, the passed fraction is optionally subjected to treatments such as concentration, desalting and the like and then subjected to the next step. When the protein having a desired property adsorbs onto the column, the initial stage eluate or an initial stage eluate by which the protein having a desired property is not eluted is mixed with a final eluate and applied to the column, and components unadsorbed onto the column are washed. Ultraviolet ray absorption of the eluted fractions is measured, and when the ultraviolet ray absorption becomes constant, the elution is carried out by a stepwise method or a gradient method using the final eluate as an eluate. A fraction containing the protein having a desired property can be detected by a usual protein detection method such as ultraviolet ray absorption, electrophoresis, activity measurement or the like.

The thus purified protein having a desired property obtained by the combination of above methods can be used as a purified bulk or as a pharmaceutical protein, by carrying out sterilization filtration after optionally adjusting protein concentration, kind of buffer, salt concentration, buffer pH and the like using an ultrafiltration (UF) membrane and gel filtration chromatography or the like.

### BEST MODE FOR CARRYING OUT THE INVENTION

### Example 1

### Modification of antibody for ganglioside GM2:

A mouse monoclonal antibody KM696 (hereinafter simply referred to as "KM696") for ganglioside GM2 belongs to an IgM type. IgM is a pentamer having an antibody basic structure composed of four chains of two L chains and two H chains. An anti-ganglioside GM2 antibody KM966 (hereinafter simply referred to as "KM966") as a mouse-human chimeric antibody was produced from the KM696 by the methods described in *Cancer Research,* 54, 1551-1516 (1994) and Japanese Published Unexamined Patent Application No. 20594/94, by substituting the H chain Fc moiety of KM696 with human Fc and substituting the L chain constant region with human κ chain.

Regarding the thus obtained KM696 and KM966, each isoelectric point of H chains and L chains of the antibody molecules were calculated by GENETYX-MAC (10.1). The values of H chains of KM696 and KM966 were 6.52 and 8.05, respectively, and the values of L chains thereof were 7.68 and 8.05, respectively. The results show that isoelectric points of the antibody molecules were changed to basic by the modification of the constant region amino acid sequence.

Although the antibodies were changed to IgG1-type antibodies after the modification of the amino acid sequence, sufficiently high hydrophobicity and binding activity for ganglioside GM2 were maintained. By the above step, it was able to produce the antibody in which the isoelectric point was modified to be basic without changing its binding activity for ganglioside GM2 and hydrophobicity.

### Example 2

### Purification of antibody KM966:

KM966-producing transformant KM966 (FERM BP-3931) was suspended in GIT medium (manufactured by Japan Pharmaceutical) containing 0.5 mg/ml G418 and 200 nmol/l MTX to give a density of 1 to 2×10⁵ cells/ml and dispensed at 100 ml into 175 cm² flasks (manufactured by Greiner). The cells were cultured at 37°C for 5 to 7 days in a CO₂ incubator, and the culture broth was recovered when they became confluent. The culture broth was mixed with the same volume of 2 mol/l ammonium sulfate-50 mmol/l sodium phosphate buffer (pH 7.0), allowed to stand for a while and then centrifuged at 8,000 rpm for 30 minutes to remove the precipitate. The supernatant was then filtered using a 0.22 µm filter (manufactured by Millipore) and the filtrate was subjected to the following step.

A resin for hydrophobic chromatography (Ether-TOYOPEARL 650M) was packed in a column manufactured by Pharmacia (XK50/250) and subjected to stationary washing with 1 N sodium hydroxide, and the column was thoroughly equilibrated with 20 mmol/l sodium phosphate buffer (pH 7.0) containing 1 M ammonium sulfate. The above filtrate was passed through the equilibrated column. After it was passed through, 20 mmol/l sodium phosphate buffer (pH 7.0) containing 1 M ammonium sulfate was passed through the column until contaminated proteins unadsorbed onto the column were thoroughly washed out (until ultraviolet ray absorption became constant). Thereafter, the antibody was eluted and fractionated by linearly decreasing the ammonium sulfate concentration in the 20 mmol/l sodium phosphate buffer containing 1 M ammonium sulfate. Fractions containing the antibody were confirmed by SDS polyacrylamide gel electrophoresis [Laemmli, *Nature,* 227, 680 (1970) (hereinafter referred to as "SDS-PAGE")] of each fraction. The antibody-containing fractions were sterilized by filtration using a 0.22 µm filter and stored at 4°C or cryopreserved at a temperature of between -40°C to -80°C until the next step.

The main fraction obtained in the above step was diluted about 10 folds with distilled water for injection (manufactured by Otsuka Pharmaceutical), and the diluted fraction was filtered using a 0.22 µm filter. The filtrate was passed through S-Sepharose HP 60/100 (manufactured by Pharmacia) column which had been subjected to stationary washing with 1 N sodium hydroxide and thoroughly equilibrated with 20 mmol/l sodium phosphate buffer in advance. After it was passed through, the column was washed with 20 mM sodium phosphate buffer until the antibody unadsorbed onto the column was not remained in the passing solution (until ultraviolet ray absorption in the passing solution became constant). Thereafter, the antibody was eluted and fractionated by linearly increasing sodium chloride concentration in the 20 mmol/l sodium phosphate buffer until the concentration became 0.4 mol/l. Fractions containing the antibody were confirmed by SDS-PAGE. The antibody-containing fractions were sterilized by filtration using a 0.22 µm filter and stored at 4°C until subjecting to the next step.

The main fraction obtained in the above step was filtered using a 0.22 µm filter. The filtrate was passed through Sephacryl S-200 (manufactured by Pharmacia) column which had been subjected to stationary washing with 1 N sodium hydroxide and then thoroughly equilibrated with 10 mmol/l sodium citrate-3.5% arginine solution in advance. Fractions in which the monomer was eluted were recovered by monitoring protein elution with ultraviolet ray absorption.

In the SDS-PAGE under non-reducing conditions, an antibody of about 150 kilodaltons having the correct size of two H chains and two L chains was purified, and under reducing conditions, the H chain having a molecular weight of about 50 kilodaltons and the L chain having a molecular weight of about 25 kilodaltons was purified. Accordingly, it was confirmed that an antibody of H chain and L chain having correct molecular weights was purified. In addition, since the purity under reducing condition was 95% or more, its purity as an antibody for medicinal use was established. Also, amounts of endotoxin and contaminated DNA were sufficiently low to be suitable for medicinal use.

### Example 3

### Modification of antibody for human interleukin-5 receptor α chain:

An antibody was modified as follows in order to obtain an antibody keeping the binding activity for human interleukin-5 receptor α chain and the activity to inhibit binding of human interleukin-5 to human interleukin-5 receptor α chain.

An anti-human interleukin-5 receptor α chain antibody KM8399 (hereinafter simply referred to as "KM8399") as a human CDR-grafted antibody was prepared by the method described in WO 97/10354, by substituting the H chain Fc moiety and the framework moiety of variable region of a mouse anti-human interleukin-5 receptor α chain monoclonal antibody KM1259 (hereinafter simply referred simply to as "KM1259"), with a human Fc and variable region framework moiety, and substituting the L chain constant region with a human κ chain.

Regarding KM1259 and KM8399 obtained by modifying KM1259, each isoelectric point of H chains and L chains of the antibody molecules were calculated by GENETYX-MAC (10.1). The values of H chains of KM1259 and KM8399 were 4.59 and 6.35, respectively, and the values of L chains were 7.63 and 8.54, respectively. The results show that the isoelectric point of the antibody molecule was changed to basic by the modification of the amino acid sequence. KM8399 maintained the binding activity for human interleukin-5 receptor α chain, the activity to inhibit binding of human interleukin-5 to human interleukin-5 receptor α chain and sufficient hydrophobicity. By the above steps, it was able to produce an antibody in which the isoelectric point was modified to basic without changing its biological activity and hydrophobicity, from the antibody for human interleukin receptor α chain.

### Example 4

### Purification of KM8399:

A KM8399-producing transformant KM8399 (FERM BP-5648) was suspended in GIT medium (manufactured by Japan Pharmaceutical) containing 0.5 mg/ml of G418 and 200 nmol/l of MTX to give a density of 1 to 2×10⁵ cells/ml and dispensed at 100 ml into 175 cm² flasks (manufactured by Greiner). The cells were cultured at 37°C for 5 to 7 days in a CO₂ incubator, and the culture broth was recovered when they became confluent. The culture broth was adjusted to pH 3 to 4, preferably 3.7, with 1 N hydrochloric acid and allowed to stand at room temperature for 1 hour to carry out inactivation of the virus. The mixture was neutralized with 1 N sodium hydroxide and then subjected to the following step.

To the mixture, 50 mmol/l sodium phosphate buffer (pH 7.0) containing 2 mol/l ammonium sulfate was added to give a final concentration of 1.1 mol/l. In the case where precipitate was formed, the mixture was centrifuged at 8,000 rpm for 30 minutes to remove the precipitate and further filtered using a 0.22 µm filter (manufactured by Millipore) to obtain a filtrate. A resin for hydrophobic chromatography (Phenyl-Sepharose 6FF lowsub) was packed in a column, subjected to stationary washing with 0.5 N sodium hydroxide and then thoroughly equilibrated with 1 mol/l ammonium sulfate-20 mmol/l sodium phosphate buffer (pH 7.0). The filtrate prepared in the above was passed through the column, and then the column was washed with 1.1 mol/l ammonium sulfate-20 mmol/l sodium phosphate buffer (pH 7.0) until ultraviolet ray absorption of unadsorbed antibody became sufficiently low. Thereafter, the antibody was eluted by linearly decreasing the ammonium sulfate concentration in the 1 mol/l ammonium sulfate-20 mmol/l sodium phosphate buffer. Fractions containing the antibody were confirmed by SDS-PAGE, and the antibody-containing fractions were sterilized by filtration using a 0.22 µm filter and stored at 4°C or cryo-preserved at a temperature of between -40°C to -80°C until subjecting to the next step.

The main fraction obtained in the above step was diluted about 15 folds with distilled water for injection and filtered using a 0.22 µm filter. The filtrate was passed through SP-Sepharose FF (manufactured by Pharmacia) column which had been subjected to stationary washing with 1 N sodium hydroxide and thoroughly equilibrated with 20 mmol/l sodium phosphate buffer (pH 7.0) in advance. After it was passed through, the column was washed with 20 mmol/l sodium phosphate buffer (pH 7.0) until the antibody unadsorbed onto the column was not remained in the passing solution (until ultraviolet ray absorption in the passing solution became constant). Thereafter, the antibody was eluted by linearly increasing sodium chloride concentration in the 20 mmol/l sodium phosphate buffer until the concentration became 0.4 mol/l. The fraction containing the antibody was confirmed by SDS-PAGE and filtered using a 0.22 µm filter, and the filtrate was passed through Sephacryl S-300 (manufactured by Pharmacia) column which had been subjected to stationary washing with 1 N sodium hydroxide and then thoroughly equilibrated with 0.9% or 9% sodium chloride solution in advance. A fraction in which the monomer was eluted was recovered by monitoring elution of protein with ultraviolet ray absorption. The purity of the fraction was confirmed by carrying out SDS-PAGE and activity measurement. In the SDS polyacrylamide gel electrophoresis [Laemmli, *Nature,* 227, 680 (1970)] under non-reducing conditions, an antibody of a correct size (about 150 kilodaltons) of two H chains and two L chains was purified, and under reducing conditions, the H chain having a molecular weight of about 50 kilodaltons and the L chain having a molecular weight of about 25 kilodaltons was purified. Accordingly, it was confirmed that an antibody of H chain and L chain having correct molecular weights was purified. In addition, since the purity under reducing condition was 95% or more, the purity as an antibody for medicinal use was established. Also, amounts of endotoxin and contaminated DNA were sufficiently low to be suitable for medicinal use.

### INDUSTRIAL APPLICABILITY

According to the present invention, a protein having a desired property can be purified efficiently and in a high yield, by separating impurities and the protein having a desired property coexisting in a solution.

## Claims

1. A method for separating and purifying a protein, which comprises:
changing a physicochemical property of a protein while having a desired property of the protein, and then
separating and purifying the protein.

2. The method according to claim 1, wherein the physicochemical property is at least one selected from an isoelectric point and hydrophobicity of the protein.

3. The method according to claim 2, wherein the isoelectric point is pI 7 or more.

4. The method according to claim 2, wherein the isoelectric point is pI 4.5 or less.

5. The method according to claim 2, wherein the hydrophobicity is similar to or higher than that of immunoglobulin G.

6. The method according to claim 2, wherein the hydrophobicity is such a degree that the protein can be adsorbed onto a phenyl sepharose resin in a buffer comprising 1 mol/l ammonium sulfate.

7. The method according to claim 2, wherein the hydrophobicity is similar to or lower than that of serum albumin.

8. The method according to claim 1, wherein the method of changing a physicochemical property of the protein is deletion, substitution or addition of an amino acid constituting the protein, or preparation of a fusion protein with other protein.

9. The method according to any one of claims 1 to 8, wherein the step of separating and purifying the protein which has a desired property comprises a step of using at least one chromatography of hydrophobic chromatography and ion exchange chromatography.

10. The method according to any one of claims 1 to 9, wherein the protein is an antibody.
